# EUROPÄISCHE PATENTSCHRIFT

(11) **EP 0 632 030 B1**
(45) Veröffentlichungstag und Bekanntmachung des Hinweises auf die Patenterteilung: **26.09.2001**
(21) Anmeldenummer: 94109699.2
(22) Anmeldetag: 23.06.1994
(51) Int. Cl.: C07D 295/02, C07D 207/26, C07D 207/32

(54) **Verfahren zur Herstellung von Pyrrolidin-Derivaten durch Pd-katalysierte Addition von Aminen an Vinyloxiranen**
Process for the preparation of pyrrolidine derivatives by Pd-catalysed addition of amines to vinyl oxiranes
Procédé pour la préparation de pyrrolidines par addition catalysée par le palladium d'amines à des vinyl oxirannes

(30) Priorität: 01.07.1993 DE 4321843
(43) Veröffentlichungstag der Anmeldung: 04.01.1995
(73) Patentinhaber: BASF Aktiengesellschaft, 67063 Ludwigshafen (DE)
(72) Erfinder: Pinkos, Rolf, Dr., D-67098 Bad Duerkheim (DE); Fischer, Rolf, Dr., D-69121 Heidelberg (DE)

(56) Entgegenhaltungen:
- US-A- 2 689 263
- JOURNAL OF THE AMERICAN CHEMICAL SOCIETY., Bd.103, 1981, GASTON, PA US Seiten 5969 - 5972 B. M. TROST, G. A. MOLANDER 'Neutral Alkylations via Palladium(0) Catalysis'
- TETRAHEDRON LETTERS., Bd.22, Nr.27, 1981, OXFORD GB Seiten 2557 - 2578 TSUJI, JIRO; KATAOKA, HIDEAKI; KOBAYASHI, YUICHI 'Regioselective 1,4-addition of nucleophiles to 1,3-diene monoepoxides catalyzed by palladium complex'
- TETRAHEDRON LETTERS., Bd.22, 1981, OXFORD GB Seiten 195 - 198 OVERMAN, LARRY E.; FLIPPIN, LEE A. 'Facile aminolysis of epoxides with diethylaluminum amides'
- THE JOURNAL OF GENRAL CHEMISTRY OF THE USSR (Z. OBSC. CHIM.), Bd.26, Nr.7, Juli 1956 Seiten 2125 - 2127 A.A. PETROV, V.M. ALBITSKAYA 'The Reaction of Vinyl Oxide with Amines.'
- SYNLETT, September 1991 Seiten 693 - 694 T. NAOTA, S.-I. MURAHASHI 'Ruthenium-Catalyzed Transformations of Amino Alcohols to Lactams.'

## Beschreibung

Die vorliegende Erfindung betrifft ein Verfahren zur Herstellung von Stickstoff-Fünfringheterocyclen durch Umsetzung von Vinyloxiranen mit Ammoniak oder primären, sekundären Aminen in Gegenwart einer Verbindung eines Elementes der VIII. oder I. Nebengruppe des Periodensystems der Elemente, und anschließende Cyclisierung in Gegenwart von Wasserstoff und eines Hydrierkatalysators bei erhöhten Temperaturen.

Aus der US-A-2 689 263 ist die Ringöffnung von Vinyloxiranen an Position 2 mit Aminen in Gegenwart von sauren Ionentauschern bekannt.

Aus Z.obsc.Chim. 2125 bis 2127 (1956) engl. Edition ist die unkatalysierte Ringöffnung von Vinyloxiranen an Position 1 bekannt.

Aus Synlett. 693 bis 694 (1991) ist die Cyclisierung der durch Ringöffnung mit Aminen erhaltenen Verbindungen mit komplexen Rutheniumhydriden in Gegenwart von Wasserstoffakzeptoren in der Flüssigphase bekannt.

Aus dem Stand der Technik laßt sich nicht ableiten, wie man aus Vinyloxiranen der Formel II Verbindungen der Formel I herstellt.

Der vorliegenden Erfindung lag daher die Aufgabe zugrunde, Vinyloxirane der Formel II in Verbindungen der Formel I umzuwandeln.

Demgemäß wurde ein neues Verfahren zur Herstellung von Stickstoff-Fünfringheterocyclen der allgemeinen Formel I 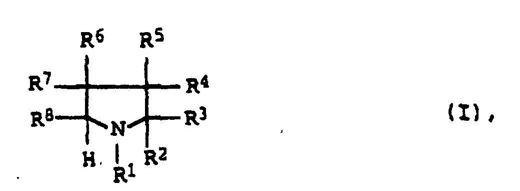 in der
- R¹,R²,R³,R⁴,R⁵,R⁶,R⁷,R⁸: Wasserstoff,
- R¹: C₁- bis C₁₀-Alkyl, C₁- bis C₁₀-Hydroxyalkyl, C₁- bis C₁₀-Aminoalkyl. Aryl, C₇- bis C₁₀-Aralkyl und C₇- bis C₁₀-Alkylaryl,
- R⁵ und R⁶: C₁- bis C₈-Alkyl,
- R² und R³: gemeinsam Sauerstoff, mit der Maßgabe, daß R⁸ für Wasserstoff steht,
- R³ und R⁴: und/oder R⁷ und R⁸ oder R⁴ und R⁷ gemeinsam eine Bindung,
bedeuten, gefunden, welches dadurch gekennzeichnet ist, daß man Vinyloxirane der allgemeinen Formel II 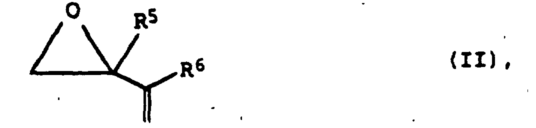 in der R⁵ und R⁶ die oben genannten Bedeutungen haben, mit Ammoniak oder primären Aminen der allgemeinen Formel III

R¹ - NH₂ (III),

in der R¹ die oben genannten Bedeutungen hat, in Gegenwart einer Verbindung eines Elementes der VIII. oder I. Nebengruppe des Periodensystems der Elemente wie in Anspruch 1 präzisiert, gegebenenfalls unter Zusatz einer Lewissäure, bei Temperaturen von 20 bis 200°C und Drücken von 1 bis 70 bar umsetzt, und anschließend in Gegenwart von Wasserstoff und eines Hydrierkatalysators wie in Anspruch 1 präzisiert bei Temperaturen von 150 bis 350°C und Drücken von 0,1 bis 300 bar cyclisiert.

Das erfindungsgemäße Verfahren kann wie folgt durchgeführt werden:

Die Vinyloxirane II und Ammoniak oder primären Amine III, gegebenenfalls in einem inerten Lösungsmittel, können mit katalytisch wirksamen Mengen einer Verbindung eines Elementes der VIII. oder I. Nebengruppe des Periodensystems der Elemente, gegebenenfalls unter Zusatz einer Lewissäure, zusammengegeben werden und beispielsweise in einem Druckgefäß bei Temperaturen von 20 bis 200°C, bevorzugt 50 bis 150°C, besonders bevorzugt 70 bis 130°C und Drücken von 1 bis 70 bar, bevorzugt 2 bis 50 bar, besonders bevorzugt 3 bis 30 bar umsetzt werden. Die Reaktionsausträge können bevorzugt aufgearbeitet werden, z.B. durch Destillation, und anschließend können die ungereinigten, bevorzugt die gereinigten Zwischenprodukte unter hydrierenden/dehydrierenden Bedingungen mit Wasserstoff, gegebenenfalls in einem inerten Lösungsmittel, in Gegenwart eines Hydrierkatalysators in der Flüssig- oder Gasphase bei Temperaturen von 150 bis 350°C, bevorzugt 180 bis 310°C, besonders bevorzugt 200 bis 300°C und Drücken von 0,1 bis 300 bar, bevorzugt 5 bis 100 bar, besonders bevorzugt 10 bis 80 bar cyclisiert werden.

Die Cyclisierung kann diskontinuierlich, z.B. in Autoklaven, oder kontinuierlich z.B. in Rohrreaktoren durchgeführt werden. Die verweilzeit betragt in der Regel zwischen 5 Minuten und 3 Stunden, bevorzugt 10 Minuten und 1,5 Stunden.

Wird die Cyclisierung in der Gasphase durchgeführt, so beträgt der Reaktionsdruck in der Regel 0,1 bis 50 bar, bevorzugt 0,5 bis 10 bar.

Als Lösungsmittel für beide Umsetzungen, die Ringöffnung und die anschließende Cyclisierung, eignen sich z.B. Wasser oder cyclische Ether wie Tetrahydrofuran oder Dioxan.

Als Katalysatoren für die Ringöffnung der Vinyloxirane II eignen sich Verbindungen der VIII. Nebengruppe, bevorzugt Palladiumverbindungen in der Oxidationsstufe Null wie Tetrakis-(triphenylphosphin)-palladium(0), Bis-[bis-(1,2-diphenylphosphino)-ethan]-palladium(0) oder Dibenzalaceton-palladium(0), besonders bevorzugt ist Tetrahis-(triphenylphosphin)-palladium (0) oder der I. Nebengruppe des Periodensystems der Elemente, bevorzugt Kupfer-I-verbindungen wie Kupfer-I-fluorid, Kupfer-I- chlorid, Kupfer-I-bromid, Kupfer-I-jodid, Kupfercyanid, besonders bevorzugt Kupfer(I)chlorid und -bromid.

Die Katalysatoren werden in der Regel in einem Gewichtsverhältnis zu den Vinyloxiranen II von 0,001 : 1 bis 0,5 : 1, bevorzugt 0,01 : 1 bis 0,1 : 1 eingesetzt.

Als Kokatalysatoren können Lewissäuren zugesetzt werden. Beispiele hierfür sind Halogenide, Sulfate oder Phosphate der I., II. und III. Hauptgruppe sowie der I., II. und III. Nebengruppen des Periodensystems der Elemente wie LiF, LiCl, CuCl₂, ZnSO₄, ZnCl₂ und Al₂(SO₄)₃.

Die Kokatalysatoren können in der Regel in einem Gewichtsverhältnis zu den Vinyloxiranen II von 0,005 : 1 bis 0,5 : 1, bevorzugt 0,01 : 1 bis 0,4 : 1 zugesetzt werden.

Bevorzugte Verbindungen III sind Ammoniak, Methylamin, Ethylamin, Ethanolamin und Anilin.

Das Molverhältnis der Verbindungen III zu den Vinyloxiranen II beträgt in der Regel 0,5 : 1 bis 150 : 1, bevorzugt 1 : 1 bis 100 : 1, besonders bevorzugt 2 : 1 bis 50 : 1.

Das Produktgemisch läßt sich z.B. destillativ aufarbeiten. Dabei kann überschüssiges Amin und gegebenenfalls nicht umgesetztes Vinyloxiran II abgetrennt und zurückgeführt werden. Verbindungen der Formel IVb und IVc 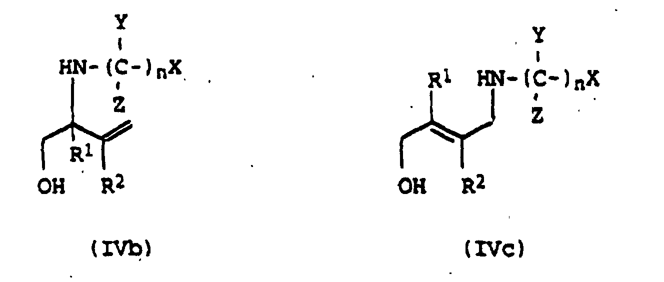 können dabei destillativ von Verbindungen der Formel IVa 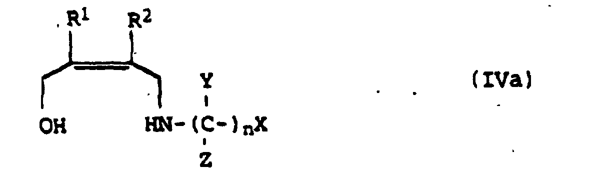 getrennt werden. Die Verbindungen der Formel IVb und c können, zur Erhöhung der Gesamtselektivität, nach bekannten Methoden in Verbindungen der Formel IVa umgelagert werden (z.B. nach Bull. Chem. Soc. France 1965, 2082 bis 2089).

Als Hydrierkatalysatoren eignen sich solche, die Ketone oder Aldehyde katalytisch mit Wasserstoff zu Alkoholen zu hydrieren vermögen. Beispiele hierfür sind in Houben/Weyl, Methoden der organischen Chemie, Band IV/1c, Georg Thieme Verlag Stuttgart, Seite 16 bis 44 (1980) beschrieben wie z.B. Elemente der I. und VI. bis VIII. Nebengruppe des Periodensystems der Elemente z.B. in Form der Metalle, ihrer Oxide und Sulfide. Sie können z.B. als Trägerkatalysatoren, Skelettkatalysatoren, Schwarzkatalysatoren oder metallische Mischkatalysatoren eingesetzt werden. Beispiele sind Pt-Schwarz, Pt/C, Pt/Al₂O₃, PtO₂, Pd-Schwarz, Pd/C, Pd/Al₂O₃, Pd/SiO₂, Pd/CaCO₃, Pd/BaSO₄, Rh/C, Rh/Al₂O₃, Ru/SiO₂, Ni/SiO₂, Raney-Nickel, Co/SiO₂, Co/Al₂O₃, Raney-Kobalt, Fe, eisenhaltige Mischkatalysatoren, Re- Schwarz, Raney-Rhenium, Cu/SiO₂, Cu/Al₂O₃, Raney-Kupfer, Cu/C, PtO₂/Rh₂O₃, Pt/Pd/C, CuCr₂O₄, BaCr₂O₄, Ni/Cr₂O₃/Al₂O₃, Re₂O₇, CoS, NiS, MoS₃, Cu/SiO₂/MoO₃/Al₂O₃. Bevorzugte Katalysatoren sind solche, die Cu als Komponente enthalten.

Die Substituenten R¹, R², R³, R⁴, R⁵, R⁶, R⁷ und R⁸ in den Verbindungen I, II, III und IVa bis c haben folgende Bedeutungen:
- R¹,R²,R³,R⁴,R⁵,R⁶,R⁷,R⁸: - Wasserstoff,
- R¹: - C₁- bis C₁₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₁- bis C₁₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methylethyl,
- C₁- bis C₁₀-Aminoalkyl, bevorzugt C₁- bis C₈-Aminoalkyl, besonders bevorzugt C₁- bis C₄-Aminoalkyl wie Aminomethyl, 1-Aminoethyl, 2-Aminoethyl, 1-Amino-n-propyl, 2-Amino-n-propyl, 3-Amino-n-propyl und 1-Amino-methyl-ethyl,
- Aryl wie Phenyl,
- C₇- bis C₁₀-Aralkyl, bevorzugt C₇- bis C₉-Phenylalkyl wie Benzyl, 1-Phenethyl, 2-Phenethyl, 1-Phenyl-propyl, 2-Phenylpropyl und 3-Phenyl-propyl, besonders bevorzugt Benzyl, 1-Phenethyl und 2-Phenethyl,
- -C₇- bis C₁₀-Alkylaryl, bevorzugt C₇- bis C₉-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- R⁵ und R⁶: - C₁- bis C₈-Alkyl, bevorzugt C₁- bis C₄-Alkyl wie Methyl, Ethyl, n-Propyl, iso-Propyl, n-Butyl, iso-Butyl, sec.-Butyl und tert.- Butyl, besonders bevorzugt Methyl und Ethyl,
- R² und R³: - gemeinsam Sauerstoff, mit der Maßgabe, daß R⁸ für Wasserstoff steht, und
- R³ und R⁴: und/oder R⁷ und R⁸ oder R⁴ und R⁷ gemeinsam eine Bindung.

Die Stickstoff-Fünfringheterocyclen eignen sich z.B. als Lösungsmittel oder Synthesebausteine für Wirkstoffsynthesen.

### Beispiele

### Beispiel 1

10 g Vinyloxiran, 25 g Methylamin und 0,6 g Pd(PPh3)4 wurden in einem Glasdruckgefäß 1 h in einem 60°C warmen Ölbad erwärmt. Der Druck in Reaktionsgefäß stieg bis auf ca. 15 bar an und betrug gegen Ende der Reaktion 9 bar. Im Reaktionsaustrag fanden sich nach Entfernen des überschüssigen Methylamins 33 % 1-Hydroxy-4-(N-methylamino)buten-2, 39 % 1-Hydroxy-2-(N-methylamino)buten-3 und 3 % 2- Hydroxy-1-(N-methylamino)buten-3.

### Beispiel 2

1 g Vinyloxiran, 4,6 g Methylamin, 0,1 g Pd(PPh3)4, 0,1 g ZnCl2 und 0,02 g LiI wurden analog Beispiel 1 umgesetzt. Im Reaktionsaustrag fanden sich nach Entfernen des überschüssigen Methylamins 30 % 1- Hydroxy-4-(N-methylamino)buten-2 und 57 % 1-Hydroxy-2-(N-methylamino)buten-3.

### Beispiel 3

Analog Beispiel 2 wurden 23 g Vinyloxiran, 107 g Methylamin, 2,7 g Pd(PPh3)4, 0,5 g LiI und 2,5 g ZnCl2 umgesetzt. Die Reaktion war nach 10 Minuten beendet. Im Reaktionsaustrag fanden sich nach Entfernen des überschüssigen Methylamins 37 % 1-Hydroxy-4-(N-methylamino)buten-2 und 52 % 1-Hydroxy-2-(N-methylamino)-buten-3. Das Austragsgemisch wurde bei einer Sumpftemperatur von 95°C bei 0,1 mbar kurzwegdestilliert. Es wurden 26 g eines farblosen Öls erhalten, welches über eine 30 cm Kolonne nochmals destilliert wurde. Bei 0,1 mbar wurden über Kopf, bei einem Kochpunkt von 39 - 45°C, 15 g 96 %iges 1- Hydroxy-2-(N-methylamino)-buten-3 erhalten. Es verblieben ca. 11 g Sumpf, welcher zu 98 % aus 1-Hydroxy-4-(N-methylamino)buten-2 bestand.

### Beispiel 4

5,5 g 1-Hydroxy-4-(N-methylamino)buten-2 und 3 g eines mit Wasserstoff aktivierten CuO (10%)/Aktivkohle-Katalysators wurden in einen 50 ml Metallautoklaven gefüllt. Nach Aufpressen von 50 bar Wasserstoff wurde 1 h auf 250°C erhitzt. Nach Abkühlen und Entspannen fanden sich im Reaktionsaustrag 50 % N-Methylpyrrolidin, 15 % N-Methylpyrrol und 1 % N-Methylpyrrolidon.

### Beispier 5

20 ml/h einer 10 gew.-%igen Lösung von 1- Hydroxy-4-(N-methylamino)buten-2 in Tetrahydrofuran wurden bei 250°C und 1013 mbar in einem Wasserstoffstrom (10 l/h) über einen Cu/SiO₂-Katalysator (11 % CuO, 7 % CaO. 1 % Na2O aud SiO₂) geleitet. Im Reaktionsaustrag fanden sich 65 % N-Methylpyrrolidin, 25 % N-Methylpyrrol und 10 % N-Methylpyrrolidon.

## Patentansprüche

1. Verfahren zur Herstellung von Stickstoff-Fünfringheterocyclen der allgemeinen Formel I 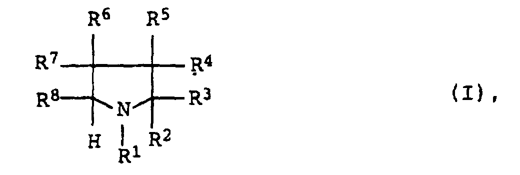 in der
R¹,R²,R³,R⁴,R⁵,R⁶,R⁷,R⁸ Wasserstoff,
R¹ C₁- bis C₁₀-Alkyl, C₁- bis C₁₀-Hydroxyalkyl, C₁- bis C₁₀-Aminoalkyl, Aryl, C₇- bis C₁₀-Aralkyl und C₇- bis C₁₀-Alkylaryl,
R⁵ und R⁶ C₁- bis C₈-Alkyl,
R² und R³ gemeinsam Sauerstoff, mit der Maßgabe, daß R⁸ für Wasserstoff steht,
R³ und R⁴ und/oder R⁷ und R⁸ oder R⁴ und R⁷ gemeinsam eine Bindung,
bedeuten, **dadurch gekennzeichnet, daß** man Vinyloxirane der allgemeinen Formel II 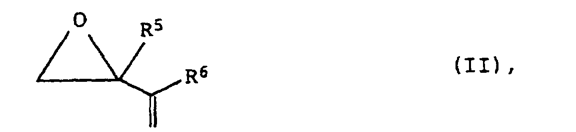 in der R⁵ und R⁶ die oben genannten Bedeutungen haben, mit Ammoniak oder primären Aminen der allgemeinen Formel III
R¹ - NH₂ (III),
in der R¹ die oben genannten Bedeutungen hat, in Gegenwart einer Komplexverbindung des Palladiums der Oxidationsstufe 0 oder eines Kupfer-I-salzes, gegebenenfalls unter Zusatz einer Lewissäure, bei Temperaturen von 20 bis 200°C und Drücken von 1 bis 70 bar umsetzt, und anschließend in Gegenwart von Wasserstoff und eines Elementes der I. oder VI. bis VIII. Nebengruppe des Periodensystems als Hydrierkatalysator bei Temperaturen von 150 bis 350°C und Drücken von 0,1 bis 300 bar cyclisiert.

2. Verfahren zur Herstellung von Stickstoff-Fünfringheterocyclen I nach Anspruch 1, **dadurch gekennzeichnet, daß** R¹ Wasserstoff oder Methyl, R², R³, R⁴, R⁵, R⁶, R⁷ und R⁸ Wasserstoff oder R² und R³ gemeinsam Sauerstoff oder R³ und R⁴ und R⁷ und R⁸ oder R⁴ und R⁷ gemeinsam eine Bindung bedeuten.

3. Verfahren zur Herstellung von Stickstoff-Fünfringheterocyclen I nach Anspruch 1, **dadurch gekennzeichnet, daß** man als Hydrierkatalysatoren Elemente der I. oder VI. bis VIII. Nebengruppe des Periodensystems in Form ihrer Metalle, Oxide oder Sulfide einsetzt.

4. Verfahren zur Herstellung von Stickstoff-Fünfringheterocyclen I nach Anspruch 1, **dadurch gekennzeichnet, daß** man als Hydrierkatalysatoren Kupfer enthaltende Katalysatoren einsetzt.

## Claims

1. A process for preparing five-membered nitrogen heterocycles of the general formula I 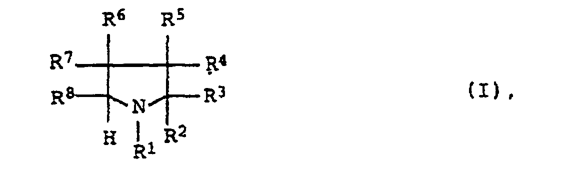 where
R¹, R², R³, R⁴, R⁵, R⁶, R⁷ and R⁸ each represent hydrogen, R¹ is also C₁-C₁₀-alkyl, C₁-C₁₀-hydroxyalkyl, C₁-C₁₀-aminoalkyl, aryl, C₇-C₁₀-aralkyl or C₇-C₁₀-alkylaryl,
R⁵ and R⁶ are C₁-C₈-alkyl
R² and R³ together represent oxygen with the proviso that R⁸ is hydrogen,
R³ and R⁴ and/or R⁷ and R⁸ or R⁴ and R⁷ together are a bond,
which process comprises reacting a vinyloxirane of the general formula II 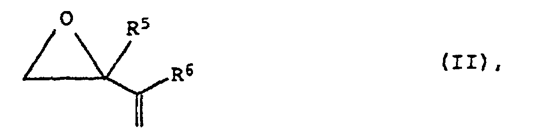 where R⁵ and R⁶ have the abovementioned meanings, with ammonia or a primary amine of the general formula III
R¹ -NH₂ (III),
where R¹ has the abovementioned meanings, in the presence of a complex compound of palladium in the zero oxidation state or a copper(I) salt, with or without the addition of a Lewis acid, at temperatures from 20 to 200°C and under pressures from 1 to 70 bar, and subsequently cyclizing in the presence of hydrogen and an element of Group I or VI to VIII or the Periodic Table as hydrogenation catalyst at temperatures from 150 to 350°C and under pressures from 0.1 to 300 bar.

2. A process for preparing five-membered nitrogen heterocycles I as claimed in claim 1, wherein R¹ is hydrogen or methyl, R², R³, R⁴, R⁵, R⁶, R⁷ and R⁸ are hydrogen or R² and R³ together are oxygen or R³ and R⁴ and R⁷ and R⁸ or R⁴ and R⁷ together are a bond.

3. A process for preparing five-membered nitrogen heterocycles I as claimed in claim 1, wherein elements of Group I or VI to VIII of the Periodic Table are employed in the form of their metals, oxides or sulfides as hydrogenation catalysts.

4. A process for preparing five-membered nitrogen heterocycles I as claimed in claim 1, wherein copper-containing catalysts are employed as hydrogenation catalysts.

## Revendications

1. Procédé de préparation d'hétérocycles azotés à maillons de formule générale I 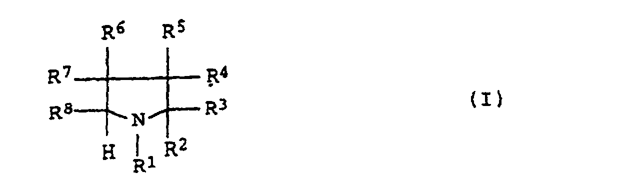 dans laquelle
R¹,R²,R³,R⁴,R⁵,R⁶,R⁷,R⁸ représentent un atome d'hydrogène,
R¹ représente un groupe alkyle en C₁ à C₁₀, hydroxyalkyle en C₁ à C₁₀, aminoalkyle en C₁ à C₁₀, aryle, aralkyle en C₇ à C₁₀, et alkylaryle en C₇ à C₁₀,
R⁵ et R⁶ représentent un groupe alkyle en C₁ à C₈,
R² et R³ représentent ensemble un atome d'oxygène, avec la condition que R⁸ représente un atome d'hydrogène,
R³ et R⁴ et/ou R⁷ et R⁸ ou R⁴ et R⁷ représentent ensemble une liaison,
**caractérisé en ce que** l'on met des vinyloxiranes de formule générale II 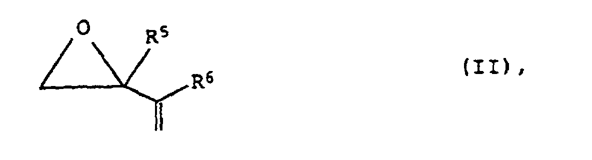 dans laquelle R⁵ et R⁶ prennent les significations précitées, à réagir avec de l'ammoniac ou des amines primaires de formule générale III
R¹ - NH₂ (III),
dans laquelle R¹ prend les significations précitées, en présence d'un composé complexe du palladium de degré d'oxydation 0 ou d'un sel de cuivre I, éventuellement avec ajout d'un acide de Lewis, à des températures de 20°C à 200°C et sous des pressions de 1 à 70 bars, puis on réalise une cyclisation en présence d'hydrogène et d'un élément du sous-groupe I ou VI jusqu'à VIII du Tableau Périodique en tant que catalyseur d'hydrogénation à des températures de 150°C à 350°C et sous des pressions de 0,1 à 300 bars.

2. Procédé de préparation d'hétérocycles I, azotés à 5 maillons selon la revendication 1, **caractérisé en ce que** R¹ représente un atome d'hydrogène ou un groupe méthyle, R², R³, R⁴, R⁵, R⁶, R⁷ et R⁸ représentent un atome d'hydrogène ou bien R² et R³ représentent ensemble un atome d'oxygène ou R³ et R⁴ et R⁷ et R⁸, ou R⁴ et R⁷ représentent ensemble une liaison.

3. Procédé de préparation d'hétérocycles I, azotés à 5 maillons selon la revendication 1, **caractérisé en ce que** l'on met en oeuvre en tant que catalyseurs d'hydrogénation, des éléments du sous-groupe I ou VI jusqu'à VIII du Tableau Périodique, sous forme de leurs métaux, oxydes ou sulfures.

4. Procédé de préparation d'hétérocycles I, azotés à 5 maillons selon la revendication 1, **caractérisé en ce que** l'on met en oeuvre des catalyseurs contenant du cuivre en tant que catalyseurs d'hydrogénation.
